# EUROPEAN PATENT SPECIFICATION

(11) **EP 2 918 679 B1**
(45) Date of publication and mention of the grant of the patent: **09.05.2018**
(21) Application number: 13852737.9
(22) Date of filing: 26.09.2013
(51) Int. Cl.: C12N 15/11, C12Q 1/68

(54) **METHOD FOR DETECTING GASTRIC POLYP AND GASTRIC CANCER USING MARKER GENE OF GASTRIC POLYP AND GASTRIC CANCER-SPECIFIC METHYLATION**
VERFAHREN ZUR ERKENNUNG VON MAGENPOLYPEN UND MAGENKARZINOMEN MIT EINEM MARKERGEN FÜR MAGENPOLYPEN- UND MAGENKARZINOMSPEZIFISCHE METHYLIERUNG
PROCÉDÉ PERMETTANT DE DÉTECTER LES POLYPES GASTRIQUES ET LE CANCER GASTRIQUE À L'AIDE D'UN GÈNE MARQUEUR DU POLYPE GASTRIQUE ET DE LA MÉTHYLATION SPÉCIFIQUE DU CANCER GASTRIQUE

(30) Priority: 07.11.2012 KR 20120125539
(43) Date of publication of application: 16.09.2015
(73) Proprietor: Genomictree, Inc., Daejeon 305-510 (KR)
(72) Inventor: AN, Sung Hwan, Daejeon 305-761 (KR); OH, Tae Jeong, Daejeon 305-721 (KR)
(74) Representative: Held, Stephan
(86) International application number: PCT/KR2013/008623
(87) International publication number: WO 2014/073785

(56) References cited:
- DE-U1- 20 121 960
- KR-A- 20110 049 430
- KR-A- 20120 055 917
- JAN K M ET AL: "Abnormal DNA methylation according to the histologic types of early gastric adenocarcinoma", HISTOPATHOLOGY, WILEY-BLACKWELL PUBLISHING LTD, GB , vol. 61, no. Supplement 1 5 September 2012 (2012-09-05), pages 76-77, XP008180352, ISSN: 0309-0167, DOI: 10.1111/J.1365-2559.2012.04359_10.X Retrieved from the Internet: URL:http://onlinelibrary.wiley.com/doi/10. 1111/j.1365-2559.2012.04359_10.x/pdf [retrieved on 2012-08-30]
- Benjamin Hoehn: "Abstract 4517: Syndecan-2 methylation is an early detection biomarker for colorectal cancer with high sensitivity and specificity in small serum sample volumes", Cancer Research, 15 April 2012 (2012-04-15), XP055274072, Retrieved from the Internet: URL:http://cancerres.aacrjournals.org/cont ent/72/8_Supplement/4517.short [retrieved on 2016-05-20]
- BAI, FEIHU ET AL.: 'Establishment and characterization of a high metastatic potential in the peritoneum for human gastric cancer by orthotopic tumor cell implantation' DIGESTIVE DISEASES AND SCIENCES vol. 52, no. 6, 2007, pages 1571 - 1578, XP019499944
- SATO, FUMIAKI ET AL.: 'CpG island hypermethylation in progression of esophageal and gastric cancer' CANCER vol. 106, no. 3, 16 December 2005, pages 483 - 493, XP055251825
- WIKSTEN, JAN-PATRIK ET AL.: 'Epithelial and stromal syndecan- expression as predictor of outcome in patients with gastric cancer' INTERNATIONAL JOURNAL OF CANCER vol. 95, no. 1, 2001, pages 1 - 6, XP055251829

## Description

### TECHNICAL FIELD

The present invention relates to the novel use of a methylated CpG island of syndecan-2 (*SDC2*; NM_002998) gene as a gastric polyp- and gastric cancer-specific methylation biomarker, and more particularly, to the use of the methylated CpG island of syndecan-2 gene as a biomarker that enables gastric polyp and gastric cancer to be diagnosed in an early stage by measuring the methylation level thereof.

### BACKGROUND ART

Even at the present time when medical science has advanced, the 5-year survival rate of cancer patients, particularly solid tumor patients (other than blood cancer patients) is less than 50%, and about 2/3 of all cancer patients are diagnosed at an advanced stage and almost all die within 2 years after cancer diagnosis. Such poor results in cancer therapy are not only the problem of therapeutic methods, but also due to the fact that it not easy to diagnose cancer at an early stage and to accurately diagnose advanced cancer and to carry out the follow-up of cancer patients after cancer therapy.

In current clinical practice, the diagnosis of cancer is confirmed by performing tissue biopsy after history taking, physical examination and clinical assessment, followed by radiographic testing and endoscopy if cancer is suspected. However, the diagnosis of cancer by the existing clinical practices is possible only when the number of cancer cells is more than a billion and the diameter of cancer is more than 1 cm. In this case, the cancer cells already have metastatic ability, and at least half thereof have already metastasized. Meanwhile, tumor markers for monitoring substances that are directly or indirectly produced from cancers are used in cancer screening, but they cause confusion due to limitations in accuracy, since up to about half thereof appear normal even in the presence of cancer, and they often appear positive even in the absence of cancer. Furthermore, the anticancer agents that are mainly used in cancer therapy have the problem that they show an effect only when the volume of cancer is small.

The reason why the diagnosis and treatment of cancer are difficult is that cancer cells are highly complex and variable. Cancer cells grow excessively and continuously, invading surrounding tissue and metastasize to distal organs leading to death. Despite the attack of an immune mechanism or anticancer therapy, cancer cells survive, continually develop, and cell groups that are most suitable for survival selectively propagate. Cancer cells are living bodies with a high degree of viability, which occur by the mutation of a large number of genes. In order that one cell is converted to a cancer cell and developed to a malignant cancer lump that is detectable in clinics, the mutation of a large number of genes must occur. Thus, in order to diagnose and treat cancer at the root, approaches at a gene level are necessary.

Recently, genetic analysis has been actively attempted to diagnose cancer. The simplest typical method is to detect the presence of ABL: BCR fusion genes (the genetic characteristic of leukemia) in blood by PCR. The method has an accuracy rate of more than 95%, and after the diagnosis and therapy of chronic myelocytic leukemia using this simple and easy genetic analysis, this method is being used for the assessment of the result and follow-up study. However, this method has a shortcoming in that it can be applied only to some blood cancers.

Furthermore, another method has been attempted, in which the presence of genes expressed by cancer cells is detected by RT-PCR and blotting, thereby diagnosing cancer cells present in blood cells. However, this method has shortcomings in that it can be applied only to some cancers, including prostate cancer and melanoma, has a high false positive rate. In addition, it is difficult to standardize detection and reading in this method, and its utility is also limited (Kopreski, M.S. et al., Clin. Cancer Res., 5:1961, 1999; Miyashiro, I. et al., Clin. Chem., 47:505, 2001) .

Recently, genetic testing that uses a DNA in serum or blood plasma has been actively attempted. This is a method of detecting a cancer-related gene that is isolated from cancer cells and released into blood and present in the form of a free DNA in serum. It is found that the concentration of DNA in serum is increased by a factor of 5-10 times in actual cancer patients as compared to that of normal persons, and such increased DNA is released mostly from cancer cells. The analysis of cancer-specific gene abnormalities, such as the mutation, deletion and functional loss of oncogenes and tumor-suppressor genes, using such DNAs isolated from cancer cells, allows the diagnosis of cancer. In this effort, there has been an active attempt to diagnose lung cancer, head and neck cancer, breast cancer, colon cancer, and liver cancer by examining the promoter methylation of mutated K-Ras oncogenes, p53 tumor-suppressor genes and p16 genes in serum, and the labeling and instability of microsatellite (Chen, X.Q. et al., Clin. Cancer Res., 5:2297, 1999; Esteller, M. et al., Cancer Res., 59:67, 1999; Sanchez-Cespedes, M. et al., Cancer Res., 60:892, 2000; Sozzi, G. et al., Clin. Cancer Res., 5:2689, 1999).

Meanwhile, in samples other than blood, the DNA of cancer cells can also be detected. A method has been attempted in which the presence of cancer cells or oncogenes in sputum or bronchoalveolar lavage of lung cancer patients is detected by a gene or antibody test (Palmisano, W.A. et al., Cancer Res., 60:5954, 2000; Sueoka, E. et al., Cancer Res., 59:1404, 1999). Additionally, other methods of detecting the presence of oncogenes in feces of colon and rectal cancer patients (Ahlquist, D.A. et al., Gastroenterol., 119:1219-27, 2000) and detecting promoter methylation abnormalities in urine and prostate fluid (Goessl, C. et al., Cancer Res., 60:5941, 2000) have been attempted. However, in order to accurately diagnose cancers that cause a large number of gene abnormalities and show various mutations characteristic of each cancer, a method in which a large number of genes are simultaneously analyzed in an accurate and automatic manner is required. However, such a method has not yet been established.

For the accurate diagnosis of cancer, it is important to detect not only a mutated gene but also a mechanism by which the mutation of this gene occurs. Previously, studies were conducted focusing on mutations in a coding sequence, i.e., micro-changes, such as point mutations, deletions and insertions, or macroscopic chromosomal abnormalities. However, in recent years, epigenetic changes were reported to be as important as these mutations, and a typical example of the epigenetic changes is the methylation of promoter CpG islands.

In the genomic DNA of mammal cells, there is the fifth base in addition to A, C, G and T, namely, 5-methylcytosine, in which a methyl group is attached to the fifth carbon of the cytosine ring (5-mC). 5-mC is always attached only to the C of a CG dinucleotide (5'-mCG-3'), which is frequently marked CpG. The C of CpG is mostly methylated by attachment with a methyl group. The methylation of this CpG inhibits a repetitive sequence in genomes, such as Alu or transposon, from being expressed. In addition, this CpG is a site where an epigenetic change in mammalian cells appears most often. The 5-mC of this CpG is naturally deaminated to T, and thus, the CpG in mammal genomes shows only 1% of frequency, which is much lower than a normal frequency (1/4 x 1/4=6.25%).

Regions in which CpG are exceptionally integrated are known as CpG islands. The CpG islands refer to sites which are 0.2-3 kb in length, and have a C+G content of more than 50% and a CpG ratio of more than 3.75%. There are about 45,000 CpG islands in the human genome, and they are mostly found in promoter regions regulating the expression of genes. Actually, the CpG islands occur in the promoters of housekeeping genes accounting for about 50% of human genes (Cross, S. et al., Curr. Opin. Gene Develop., 5:309, 1995). Recently, an attempt to examine the promoter methylation of tumor-related genes in blood, sputum, saliva, feces or urine and to use the examined results for the diagnosis and treatment of various cancers, has been actively conducted (Esteller, M. et al., Cancer Res., 59:67, 1999; Sanchez-Cespedez, M. et al., Cancer Res., 60:892, 2000; Ahlquist, D.A. et al., Gastroenterol., 119:1219, 2000). Thus, the present inventors have demonstrated that Syndecan 2 gene can be used specifically to diagnose colon cancer based on relevant studies (KR 10-1142131 B). However, this document does not suggest the use of Syndecan 2 gene for diagnosis of other cancers including gastric cancer. Meanwhile, the present inventors have found that Syndecan 2 gene is not appropriate as a biomarker of various solid cancers such as lung cancer, breast cancer and the like, and thus it acts as only a colon cancer-specific biomarker, rather than a biomarker for diagnosis of general cancers.

Jan et al. (Jan, K. M. et al., Histopathology, 61:76, 2012) describe different genes that are hypermethylated in gastric cancer samples. Moreover, this publication describes the use of a 450 Infiunium beadchip. This chip comprises probes, which are extended as primers, leading to a linear amplification of the target molecules. The chip also comprises probes for the SDC2 CpG island, however, other genes of the chip are used as markers for the diagnosis of gastric cancers.

Hoehn et al. (Hoehn, B. et al., Canc. Res., Abstract 4517, 2012) and KR 2011 0049430 A link the methylation of SDC2 to colon cancer and DE 201 21 960 U1 links the methylation of SDC2 to cancer metastasis.

KR 2012 0055917 A and Sato et al. (Sato, F. et al., Cancer, 106:483, 2005) describe various further gastric cancer methylation markers.

Accordingly, the present inventors have made extensive efforts to develop an effective gastric-cancer-specific methylation marker which makes it possible to diagnose cancer and the risk of carcinogenesis at an early stage and predict cancer prognosis. As a result, the present inventors have found that Syndecan 2 (*SDC2*; NM_002998) gene is methylated specifically in gastric polyps and gastric cancer cells, but not in lung cancer tissue, breast cancer tissue, liver cancer tissue, cervical cancer tissue, and thyroid cancer tissue and that gastric polyps and gastric cancer can be diagnosed at an early stage by measuring the methylation level thereof using the *SDC22* gene as a biomarker, thereby completing the present invention.

The above information disclosed in this Background section is only for enhancement of understanding of the background of the present invention, and therefore it may contain information that does not form the prior art that is already known to a person of ordinary skill in the art.

### DISCLOSURE OF INVENTION

### TECHNICAL PROBLEM

It is a main object of the present invention to provide a gastric polyp- or gastric cancer-specific methylation biomarker, which is methylated specifically in gastric polyp or gastric cancer, and thus can be effectively used for the diagnosis of gastric polyp or gastric cancer, and the use thereof for providing information for early diagnosis of gastric cancer.

Disclosed is further a method for detecting methylation of the *SDC2* gene that is a gastric polyp- or gastric cancer-specific methylation marker gene, and a kit and nucleic acid chip for diagnosing gastric polyp or gastric cancer using the *SDC2* gene.

### TECHNICAL SOLUTION

To achieve the above objects, the present invention provides a use of a methylated CpG island of syndecan-2 (*SDC2*) gene located in a region set forth in SEQ ID NO: 1 for diagnosing gastric cancer or gastric polyp. Disclosed is further a kit for diagnosing gastric polyp or gastric cancer, which comprises: a PCR primer pair for amplifying a fragment comprising the CpG island of syndecan-2 (*SDC2*) gene; and a sequencing primer for pyrosequencing a PCR product amplified by the primer pair.

Disclosed is yet further a composition for diagnosing gastric polyp or gastric cancer, which contains a substance capable of detecting methylation of the CpG island of syndecan-2 (*SDC2*) gene.

Disclosed is yet further a method for detecting gastric polyp or gastric cancer, comprising the steps of:
(a) isolating DNA from a clinical sample; and
(b) measuring the methylation of the CpG island of *SDC2* (syndecan-2) gene, which is a gastric polyp or gastric cancer biomarker, in the isolated DNA to detect gastric polyp or gastric cancer.

Disclosed is yet further a composition for diagnosing gastric polyp or gastric cancer, which contains a substance capable of detecting methylation of the CpG island of the syndecan-2 (*SDC2*) gene.

Disclosed is yet further a nucleic acid chip for diagnosing gastric polyp or gastric cancer, which has immobilized thereon a probe capable of hybridizing to a fragment comprising the CpG island of syndecan-2 (*SDC2*) gene under a strict condition.

Other features and embodiments of the present invention will be more apparent from the following detailed descriptions and the appended claims.

### BRIEF DESCRIPTION OF THE DRAWINGS

FIG. 1 is a graph showing the results of measuring the methylation levels of *SDC2* biomarker gene, which binds specifically to bisphenol A, in normal gastric tissue, low-grade dysplasia and high-grade dysplasia by pyrosequencing.
FIG. 2A is a graph showing the results of measuring the methylation level of *SDC2* biomarker gene in a gastric cancer cell line by pyrosequencing; FIG. 2B is a graph showing the results of measuring the methylation levels of *SDC2* biomarker gene in normal gastric tissue, gastric cancer tissue and a normal gastric tissue adjacent to gastric cancer tissue by pyrosequencing; and FIG. 2C shows the results of measuring the sensitivity and specificity of *SDC2* biomarker gene for gastric cancer diagnosis by ROC curve analysis.
FIG. 3A shows the results of measuring the methylation levels of *SDC2* biomarker gene in the serum DNAs of normal persons and gastric cancer patients by the qMSP method; and FIG. 3B shows the results of measuring the sensitivity and specificity of *SDC2* biomarker gene for gastric cancer diagnosis by ROC curve analysis in order to evaluate the ability of the *SDC2* biomarker gene to diagnose gastric cancer.
FIG. 4 shows the results of measuring the methylation levels of *SDC2* biomarker gene in the cancer tissues and normal tissues of lung cancer, breast cancer, liver cancer, cervical cancer and prostate cancer patients by pyrosequencing.
FIG. 5 shows the results of measuring the methylation levels of *SDC2* biomarker gene in the sera of normal persons determined to be normal by gastroscopy and gastric polyp patients, and the results of analyzing the ROC curve.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein are well known and are commonly employed in the art.

The present invention is directed to the use of the CpG island of syndecan-2 (*SDC2*) gene, which is methylated specifically in gastric polyp or gastric cancer, as a biomarker. Accordingly, in one aspect, the present invention is directed to the use of a methylated CpG island of syndecan-2 (*SDC2*) gene located in a region set forth in SEQ ID NO: 1 for diagnosing gastric cancer or gastric polyp.

In the present invention, the CpG island is located in the promoter region of the *SDC2* gene, in particular, the CpG island is located in a region represented by SEQ ID NO: 1.

The gastric polyp- or gastric cancer-specific methylation marker genes can be used for gastric cancer screening, risk-assessment, prognosis, disease identification, the diagnosis of disease stages, and the selection of therapeutic targets. Particularly, the SDC2 gene that is a biomarker gene according to the present invention showed a high level and frequency of positive methylation in gastric polyp that is a precancerous lesion of gastric cancer, suggesting that the SDC2 gene is useful as a biomarker for diagnosis of gastric polyp and early diagnosis of gastric cancer.

In the present invention, the methylated CpG island can be detected by a substance capable of detecting methylation of the CpG island, which may be any one selected from the group consisting of a primer pair capable of amplifying a fragment comprising the methylated CpG island, a probe capable of hybridizing to the methylated CpG island, a methylation-specific binding protein or a methylation-specific binding antibody which is capable of binding to the methylated CpG island, a sequencing primer, a sequencing-by-synthesis primer, and a sequencing-by-ligation primer.

The identification of genes that are methylated in gastric cancer and abnormalities at various stages of gastric cancer makes it possible to diagnose gastric cancer at an early stage in an accurate and effective manner and allows methylation profiling of multiple genes and the identification of new targets for therapeutic intervention. Furthermore, the methylation data may be combined with other non-methylation related biomarker detection methods to obtain a more accurate system for gastric cancer diagnosis.

According to the herein disclosed method , the progression of gastric cancer at various stages or phases can be diagnosed by determining the methylation stage of one or more nucleic acid biomarkers obtained from a sample. By comparing the methylation stage of a nucleic acid isolated from a sample at each stage of gastric cancer with the methylation stage of one or more nucleic acids isolated from a sample in which there is no cell proliferative disorder of gastric tissue, a specific stage of gastric cancer in the sample can be detected. Herein, the methylation stage may be hypermethylation.

In one embodiment, nucleic acid may be methylated in the regulatory region of a gene. In another embodiment, a gene which is involved in cell transformation can be diagnosed by detecting methylation outside of the regulatory region of the gene, because methylation proceeds inwards from the outside of the gene.

One example of the kit disclosed herein includes: a carrier means compartmentalized to receive a sample therein; and one or more containers comprising a first container containing a reagent which sensitively cleaves unmethylated cytosine, a second container containing primers for amplification of a CpG-containing nucleic acid, and a third container containing a means to detect the presence of cleaved or uncleaved nucleic acid. Primers contemplated for use in accordance with the invention include the sequences set forth in SEQ ID NOS: 2, 3, 5 and 6, and any functional combination and fragments thereof. The functional combination or fragment is used as a primer to detect whether methylation has occurred on the region of the genome sought to be detected.

In addition, a cellular proliferative disorder (dysplasia) of gastric tissue in a sample can be diagnosed by detecting methylation of the SDC2 (syndecan-2) gene using a kit or nucleic acid chip.

Accordingly, in another aspect, the present invention is directed to the use of a modified nucleic acid for diagnosis of gastric polyp or gastric cancer, derived from a SDC2 (syndecan-2) gene fragment set forth in SEQ ID NO: 1, in which the modified nucleic acid is obtained by modifying the SDC2 gene fragment so that at least one cytosine residue in the SDC2 gene fragment will be modified to uracil or a nucleotide other than cytosine in a hybridization process.

In the present invention, the modified nucleic acid may comprise a sequence set forth in SEQ ID NO: 23.

Disclosed is further a kit for diagnosing gastric polyp or gastric cancer, which comprises: a PCR primer pair for amplifying a fragment comprising the CpG island of syndecan-2 (*SDC2*) gene; and a sequencing primer for pyrosequencing a PCR product amplified by the primer pair.

Disclosed is further a nucleic acid chip for diagnosing gastric polyp or gastric cancer, which has immobilized thereon a probe capable of hybridizing to a fragment comprising the CpG island of syndecan-2 (*SDC2*) gene under a strict condition.

The use of the diagnostic kit or nucleic acid chip allows for the detection of a cellular proliferative disorder (dysplasia) of gastric tissue in a sample. The detection method comprises determining the methylation state of at least one nucleic acid isolated from a sample, and the methylation state of the at least one nucleic acid may be compared with the methylation state of a nucleic acid isolated from a sample in which there is no cellular proliferative disorder (dysplasia) of gastric tissue.

In yet another embodiment of the present invention, cells that are likely to form gastric cancer can be diagnosed at an early stage using the methylation marker genes. When genes confirmed to be methylated in cancer cells are methylated in cells that appear normal clinically or morphologically, this indicates that the normally appearing cells progress to cancer. Thus, gastric cancer can be diagnosed at an early stage by detecting the methylation of gastric cancer-specific genes in cells that appear normally. Particularly, in an example of the present invention, it was found that the SDC2 (syndecan-2) gene can be used for diagnosis of gastric polyp that is a precancerous lesion of gastric cancer.

The present invention enables, cells that are likely to form gastric cancer, to be diagnosed at an early stage using the methylation marker genes. When genes confirmed to be methylated in cancer cells are methylated in cells that appear normal clinically or morphologically, this indicates that the normally appearing cells progress to cancer. Thus, gastric cancer can be diagnosed at an early stage by detecting the methylation of gastric cancer-specific genes in cells that appear normally.

The use of the methylation marker gene of the present invention allows for detection of a cellular proliferative disorder (dysplasia) of gastric tissue in a sample. The detection method comprises bringing a sample comprising at least one nucleic acid isolated from a subject into contact with at least one agent capable of determining the methylation state of the nucleic acid. The method comprises detecting the methylation of at least one region in at least one nucleic acid, wherein the methylation of the nucleic acid differs from the methylation state of the same region of a nucleic acid present in a sample in which there is no abnormal growth (dysplastic progression) of gastric cells.

The likelihood of progression of tissue to gastric cancer can be evaluated by examining the methylation frequency of a gene which is specifically methylated in gastric cancer, and determining the methylation frequency of tissue that is likely to progress to gastric cancer.

Therefore, in still another aspect, the present specification discloses a method for detecting gastric polyp or gastric cancer, comprising the steps of:
(a) isolating DNA from a clinical sample; and
(b) measuring the methylation of the CpG island of *SDC2* (syndecan-2) gene, which is a gastric polyp or gastric cancer biomarker, in the isolated DNA to detect gastric polyp or gastric cancer.

In this method, step (b) may be performed by measuring the methylation of any one of the promoter, 5-untranslated region (UTR), intron and exon regions of the gene. Preferably, the methylation of the CpG island in a region of the *SDC2* gene, which has a nucleotide sequence of SEQ ID NO: 1 is measured.

In the method, step (b) may be performed by a method selected from the group consisting of PCR, methylation-specific PCR, real-time methylation-specific PCR, PCR assay using a methylation DNA-specific binding protein, quantitative PCR, DNA chip-based assay, pyrosequencing, and bisulfate sequencing. In addition, the clinical sample may be selected from the group consisting of a tissue, cell, blood, blood plasma, feces, and urine from a patient suspected of cancer or a subject to be diagnosed, but is not limited thereto.

The herein disclosed method for detecting the methylation of a gene may comprise: (a) preparing a clinical sample containing DNA; (b) isolating DNA from the clinical sample; (c) amplifying the isolated DNA using primers capable of amplifying a fragment comprising the CpG island of the promoter or intron of an *SDC2* gene; and (d) determining whether the intron was methylated based on whether the DNA was amplified in step (c).

A cellular proliferative disorder (dysplasia) of gastric tissue in a sample can be diagnosed by detecting the methylation state of the *SDC2* (NM_002998, Syndecan 2) gene using a kit.

A kit for diagnosing gastric polyp or gastric cancer is disclosed, which contains: a PCR primer pair for amplifying a fragment comprising either the CpG island of the *SDC2* (NM_002998, Syndecan 2) gene; and a sequencing primer for pyrosequencing a PCR product amplified by the primer pair:
In the present invention, the PCR primer pair may be a primer pair set forth in SEQ ID NO: 2 and 3, or a primer pair set forth in SEQ ID NOS: 5 and 6.

In the present invention, the sequencing primer may be a primer set forth in SEQ ID NO: 4 or 7.

A cellular proliferative disorder (dysplasia) of gastric tissue cells in a sample can be diagnosed by detecting the methylation state of the *SDC2* (NM_002998, Syndecan 2) gene using a nucleic acid chip.

Disclosed is thus a nucleic acid chip for diagnosing gastric polyp or gastric cancer, which has immobilized thereon a probe capable of hybridizing to a fragment comprising the CpG island of syndecan-2 (*SDC2*) gene under a strict condition.

In the present invention, the methylated CpG island can be detected by a probe capable of hybridizing to a fragment comprising the CpG island. The probe may be selected from the group consisting of the base sequences set forth in SEQ ID NOS: 8 to 19, and specific examples thereof are as follows:

### SDC2

1) 5'-cggagctgcc aatcggcgtg taatcctgta-3' (SEQ ID NO: 8)
2) 5'-ctgccgtagc tccctttcaa gccagcgaat ttattcctta aaaccagaaa-3' (SEQ ID NO: 9)
3) 5'-gcacgggaaa ggagtccgcg gaggagcaaa accacagcag agcaagaaga-3' (SEQ ID NO: 10)
4) 5'-gcagccttcc cggagcacca actccgtgtc gggagtgcag aaaccaacaa gtgagagggc-3' (SEQ ID NO: 11)
5) 5'-cccgagcccg agtccccgag cctgagccgc aatcgctgcg gtactctgct-3' (SEQ ID NO: 12)
6) 5'-cttggtggcc tgcgtgtcgg cggagtcggt gagtgggcca-3' (SEQ ID NO: 13)

### Modified nucleic acid sequence probe

1') 5'-cggagttgtt aatcggcgtg taattttgta-3' (SEQ ID NO: 14)
2') 5'-ttgtcgtagt ttttttttaa gttagcgaat ttatttttta aaattagaaa-3' (SEQ ID NO: 15)
3') 5'-gtacgggaaa ggagttcgcg gaggagtaaa attatagtag agtaagaaga-3' (SEQ ID NO: 16)
4') 5'-gtagtttttt cggagtatta atttcgtgtc gggagtgtag aaattaataa gtgagagggt-3' (SEQ ID NO: 17)
5') 5'-ttcgagttcg agttttcgag tttgagtcgt aatcgttgcg gtattttgtt-3' (SEQ ID NO: 18)
6') 5'-tttggtggtt tgcgtgtcgg cggagtcggt gagtgggtta-3' (SEQ ID NO: 19)

The use of the diagnostic kit or nucleic acid chip disclosed makes it possible to determine the abnormal growth (dysplastic progression) of gastric tissue cells in a sample. The method comprises determining the methylation state of at least one nucleic acid isolated from a sample, wherein the methylation state of the at least one nucleic acid is compared with the methylation stage of a nucleic acid isolated from a sample in which there is no abnormal growth (dysplastic progression) of gastric cells.

Transformed gastric cancer cells can be detected by examining the methylation of the marker gene using the disclosed kit or nucleic acid chip.

Gastric cancer can be diagnosed by examining the methylation of the marker gene using the disclosed kit or nucleic acid chip.

The likelihood of progression to gastric cancer can be diagnosed by examining the methylation of the marker gene in a sample showing a normal phenotype using the disclosed kit or nucleic acid chip. The sample that is used may be solid or liquid tissue, cells, feces, urine, serum, or blood plasma.

Major terms which are used herein are defined as follows.

As used herein, the term "cell transformation" refers to the change in characteristics of a cell from one form to another form such as from normal to abnormal, non-tumorous to tumorous, undifferentiated to differentiated, stem cell to non-stem cell. In addition, the transformation can be recognized by the morphology, phenotype, biochemical characteristics and the like of a cell.

As used herein, the term "early detection" of cancer refers to discovering the likelihood of cancer prior to metastasis, and preferably before observation of a morphological change in a tissue or cell. Furthermore, the term "early detection" of cell transformation refers to the high probability of a cell to undergo transformation in its early stages before the cell is morphologically designated as being transformed.

As used herein, the term "hypermethylation" refers to the methylation of a CpG island.

As used herein, the term "sample" or "clinical sample" is referred to in its broadest sense, and includes any biological sample obtained from an individual, body fluid, a cell line, a tissue culture, depending on the type of assay that is to be performed. Methods for obtaining tissue biopsies and body fluids from mammals are well known in the art. A gastric tissue biopsy of the is a preferred source.

Use of cancer cells for comparison between gastric cancer biomarker and normal cells

In the present invention, "normal" cells refer to those that do not show any abnormal morphological or cytological changes. "Tumor" cells are cancer cells. "Non-tumor" cells are those cells that are part of the diseased tissue but are not considered to be the tumor portion.

The present invention is based on the discovery of the relationship between gastric cancer and the hypermethylation of *SDC2* (NM_002998, Syndecan 2) gene.

A cellular proliferative disorder of gastric tissue cell can be diagnosed at an early stage by determining the methylation stage of at least one nucleic acid from a subject using the disclosed kit or nucleic acid chip. Herein, the methylation stage of the at least one nucleic acid may be compared with the methylation state of at least one nucleic acid isolated from a subject not having a cellular proliferative disorder of gastric tissue. The nucleic acid is preferably a CpG-containing nucleic acid such as a CpG island.

A cellular proliferative disorder of gastric tissue can be diagnosed by determining the methylation of at least one nucleic acid from a subject using the kit or nucleic acid chip. Herein, the nucleic acid may be *SDC2* (NM_002998, Syndecan 2). The methylation of the at least one nucleic acid may be compared with the methylation state of at least one nucleic acid isolated from a subject having no predisposition to a cellular proliferative disorder of gastric tissue.

As used herein, "predisposition" refers to the property of being susceptible to a cellular proliferative disorder. A subject having a predisposition to a cellular proliferative disorder has no cellular proliferative disorder, but is a subject having an increased likelihood of having a cellular proliferative disorder.

In another aspect, the present specification discloses a method for diagnosing a cellular proliferative disorder of gastric tissue, the method comprising brining a sample comprising a nucleic acid into contact with an agent capable of determining the methylation state of the sample, and determining the methylation of at least one region of the at least one nucleic acid. Herein, the methylation of the at least one region in the at least one nucleic acid differs from the methylation stage of the same region in a nucleic acid present in a subject in which there is no abnormal growth of cells.

The method disclosed comprises a step of determining the methylation of at least one region of at least one nucleic acid isolated from a subject.

The term "nucleic acid" or "nucleic acid sequence" as used herein refers to an oligonucleotide, nucleotide or polynucleotide, or fragments thereof, or single-stranded or double-stranded DNA or RNA of genomic or synthetic origin, sense- or antisense-strand DNA or RNA of genomic or synthetic origin, peptide nucleic acid (PNA), or any DNA-like or RNA-like material of natural or synthetic origin. It will apparent to those of skill in the art that, when the nucleic acid is RNA, the deoxynucleotides A, G, C, and T are replaced by the ribonucleotides A, G, C, and U, respectively.

Any nucleic acid may be used in the present invention, given the presence of differently methylated CpG islands can be detected therein. The CpG island is a CpG-rich region in a nucleic acid sequence.

### Methylation

In the present invention, any nucleic acid sample, in purified or nonpurified form, can be used, provided it contains or is suspected of containing a nucleic acid sequence containing a target locus (e.g., CpG-containing nucleic acid). One nucleic acid region capable of being differentially methylated is a CpG island, a sequence of nucleic acid with an increased density relative to other nucleic acid regions of the dinucleotide CpG. The CpG doublet occurs in vertebrate DNA at only about 20% of the frequency that would be expected from the proportion of G*C base pairs. In certain regions, the density of CpG doublets reaches the predicted value; it is increased by ten-fold relative to the rest of the genome. CpG islands have an average G*C content of about 60%, compared with the 40% average in bulk DNA. The islands take the form of stretches of DNA typically about one to two kilobases long. There are about 45,000 islands in the human genome.

In many genes, the CpG islands begin just upstream of a promoter and extend downstream into the transcribed region. Methylation of a CpG island at a promoter usually suppresses expression of the gene. The islands can also surround the 5' region of the coding region of the gene as well as the 3' region of the coding region. Thus, CpG islands can be found in multiple regions of a nucleic acid sequence including upstream of coding sequences in a regulatory region including a promoter region, in the coding regions (e.g., exons), downstream of coding regions in, for example, enhancer regions, and in introns.

Typically, the CpG-containing nucleic acid is DNA. However, the above described method may employ, for example, samples that contain DNA, or DNA and RNA containing mRNA, wherein DNA or RNA may be single-stranded or double-stranded, or a DNA-RNA hybrid may be included in the sample.

A mixture of nucleic acids may also be used. The specific nucleic acid sequence to be detected may be a fraction of a larger molecule or can be present initially as a discrete molecule, so that the specific sequence constitutes the entire nucleic acid. It is not necessary that the sequence to be studied be present initially in a pure form; the nucleic acid may be a minor fraction of a complex mixture, such as contained in whole human DNA. Nucleic acids contained in a sample used for detection of methylated CpG islands may be extracted by a variety of techniques such as that described by Sambrook, et al. (Molecular Cloning: A Laboratory Manual, Cold Spring Harbor, N.Y., 1989).

Nucleic acids isolated from a subject are obtained in a biological sample from the subject. If it is desired to detect gastric cancer or stages of gastric cancer progression, the nucleic acid may be isolated from gastric tissue by scraping or biopsy. Such samples may be obtained by various medical procedures known to those of skill in the art.

In one aspect of the invention, the state of methylation in nucleic acids of the sample obtained from a subject is hypermethylation compared with the same regions of the nucleic acid in a subject not having gastric cancer or gastric polyp. Hypermethylation as used herein refers to the presence of methylated alleles in one or more nucleic acids. Nucleic acids from a subject not having gastric cancer or gastric polyp contain no detectable methylated alleles when the same nucleic acids are examined.

### Individual genes and panel

It is understood that the present invention may be practiced using the SDC2 gene separately as a diagnostic or prognostic marker or SDC2 and additional few marker genes combined into a panel display format so that several marker genes may be detected for overall pattern or listing of genes that are methylated to increase reliability and efficiency. Furthermore, the SDC2 gene identified in the present invention may be used individually or as a set of genes in any combination with any of the other genes that are recited herein. Alternatively, genes may be ranked according to their importance and weighted and together with the number of genes that are methylated, a level of likelihood of developing cancer may be assigned.

### Method for detection of methylation

Disclosed herein is a method for diagnosing gastric polyp or gastric cancer, which comprises treating genomic DNA, isolated from a clinical sample, with bisulfite to convert the SDC2 CpG site, and detecting the methylation of the converted SDC2 CpG site using at least one synthetic oligonucleotide which is complementary thereto or is capable of hybridizing thereto.

### Methylation-specific PCR

When genomic DNA is treated with bisulfite, cytosine in the 5'-CpG'-3 region remains intact, if it was methylated, but the cytosine changes to uracil, if it was unmethylated. Accordingly, based on the base sequence converted after bisulfite treatment, PCR primer sets corresponding to a region having the 5'-CpG-3' base sequence are constructed. Herein, the constructed primer sets are two kinds of primer sets: a primer set corresponding to the methylated base sequence, and a primer set corresponding to the unmethylated base sequence. When genomic DNA is converted with bisulfite and then amplified by PCR using the above two kinds of primer sets, the PCR product is detected in the PCR mixture employing the primers corresponding to the methylated base sequence, if the genomic DNA was methylated, but the genomic DNA is detected in the PCR mixture employing the primers corresponding to the unmethylated, if the genomic DNA was unmethylated. This methylation can be quantitatively analyzed by agarose gel electrophoresis.

### Real-time methylation specific PCR

Real-time methylation-specific PCR is a real-time measurement method modified from the methylation-specific PCR method and comprises treating genomic DNA with bisulfite, designing PCR primers corresponding to the methylated base sequence, and performing real-time PCR using the primers. Methods of detecting the methylation of the genomic DNA include two methods: a method of detection using a TanMan probe complementary to the amplified base sequence; and a method of detection using Sybergreen. Thus, the real-time methylation-specific PCR allows selective quantitative analysis of methylated DNA. Herein, a standard curve is plotted using an in vitro methylated DNA sample, and a gene containing no 5'-CpG-3' sequence in the base sequence is also amplified as a negative control group for standardization to quantitatively analyze the degree of methylation.

### Pyrosequencing

The pyrosequencing method is a quantitative real-time sequencing method modified from the bisulfite sequencing method. Similarly to bisulfite sequencing, genomic DNA is converted by bisulfite treatment, and then, PCR primers corresponding to a region containing no 5'-CpG-3' base sequence are constructed. Specifically, the genomic DNA is treated with bisulfite, amplified using the PCR primers, and then subjected to real-time base sequence analysis using a sequencing primer. The degree of methylation is expressed as a methylation index by analyzing the amounts of cytosine and thymine in the 5'-CpG-3' region.

### PCR Using Methylated DNA-specific binding protein, quantitative PCR, And DNA Chip Assay

When a protein binding specifically only to methylated DNA is mixed with DNA, the protein binds specifically only to the methylated DNA. Thus, either PCR using a methylation-specific binding protein or a DNA chip assay allows selective isolation of only methylated DNA. Genomic DNA is mixed with a methylation-specific binding protein, and then only methylated DNA was selectively isolated. The isolated DNA is amplified using PCR primers corresponding to the promoter region, and then methylation of the DNA is measured by agarose gel electrophoresis.

In addition, methylation of DNA can also be measured by a quantitative PCR method, and methylated DNA isolated with a methylated DNA-specific binding protein can be labeled with a fluorescent probe and hybridized to a DNA chip containing complementary probes, thereby measuring methylation of the DNA. Herein, the methylated DNA-specific binding protein may be, but not limited to, MBD2bt (truncated methyl CpG binding domain protein 2).

### Detection of Differential Methylation-Methylation-Sensitive Restriction Endonuclease

Detection of differential methylation can be accomplished by bringing a nucleic acid sample into contact with a methylation-sensitive restriction endonuclease that cleaves only unmethylated CpG sites.

In a separate reaction, the sample is further brought into contact with an isoschizomer of the methylation-sensitive restriction enzyme that cleaves both methylated and unmethylated CpG-sites, thereby cleaving the methylated nucleic acid.

Specific primers are added to the nucleic acid sample, and the nucleic acid is amplified by any conventional method. The presence of an amplified product in the sample treated with the methylation-sensitive restriction enzyme but absence of an amplified product in the sample treated with the isoschizomer of the methylation-sensitive restriction enzyme indicates that methylation has occurred at the nucleic acid region assayed. However, the absence of an amplified product in the sample treated with the methylation-sensitive restriction enzyme together with the absence of an amplified product in the sample treated with the isoschizomer of the methylation-sensitive restriction enzyme indicates that no methylation has occurred at the nucleic acid region assayed.

As used herein, the term "methylation-sensitive restriction enzyme" refers to a restriction enzyme (e.g., *Sma*I) that includes CG as part of its recognition site and has activity when the C is methylated as compared to when the C is not methylated. Non-limiting examples of methylation-sensitive restriction enzymes include *Msp*I*, Hpa*II*, Bss*HII, BstUI and NotI. Such enzymes can be used alone or in combination. Examples of other methylation-sensitive restriction enzymes include, but are not limited to SacII and *Eag*I*.*

The isoschizomer of the methylation-sensitive restriction enzyme is a restriction enzyme that recognizes the same recognition site as the methylation-sensitive restriction enzyme but cleaves both methylated and unmethylated CGs. An example thereof includes MspI.

Primers for use in the present invention are designed to be "substantially" complementary to each strand of the locus to be amplified and include the appropriate G or C nucleotides as discussed above. This means that the primers must be sufficiently complementary to hybridize with their respective strands under polymerization reaction conditions. Primers for use in the present invention are used in the amplification process, which is an enzymatic chain reaction (e.g., PCR) in which that a target locus exponentially increases through a number of reaction steps. Typically, one primer is homologous with the negative (-) strand of the locus (antisense primer), and the other primer is homologous with the positive (+) strand (sense primer). After the primers have been annealed to denatured nucleic acid, the nucleic acid chain is extended by an enzyme such as DNA Polymerase I (Klenow), and reactants such as nucleotides, and, as a result, + and - strands containing the target locus sequence are newly synthesized. When the newly synthesized target locus is used as a template and subjected to repeated cycles of denaturing, primer annealing, and extension, exponential synthesis of the target locus sequence occurs. The resulting reaction product is a discrete nucleic acid duplex with termini corresponding to the ends of specific primers employed.

The amplification reaction is PCR which is commonly used in the art. However, alternative methods such as real-time PCR or linear amplification using isothermal enzyme may also be used. In addition, multiplex amplification reactions may also be used.

### Bisulfite sequencing method

Another method for detecting a methylated CpG-containing nucleic acid comprises bringing a nucleic acid-containing sample into contact with a reagent that modifies unmethylated cytosine, and amplifying the CpG-containing nucleic acid in the sample using methylation-independent oligonucleotide primers. Herein, the oligonucleotide primers amplify nucleic acid without distinguishing between modified methylated nucleic acid and unmethylated nucleic acid. The amplified product is sequenced by the Sanger method using a sequencing primer or sequenced by a next-generation sequencing method described in relation to bisulfite sequencing for detection of methylated nucleic acid. Herein, the next-generation sequencing method may be performed by a sequencing-by-synthesis or sequencing-by-ligation technique. This method is characterized in that, instead of preparing bacterial clones, a single DNA fragment is isolated spatially, amplified *in situ* (clonal amplification), and sequenced. Herein, hundreds of thousands of fragments are read out at the same time, and for this reason, the method is also called "massively parallel sequencing method".

Typically, the sequencing-by-synthesis method is used in which signals are obtained while mono- or di-nucleotides are sequentially added. Examples of this method include pyrosequencing, ion torrent, and Solexa methods.

NGS systems based on sequencing-by-synthesis include 454 platform (Roche), HiSeq platform (Illumina), Ion PGM platform (Life Technology), and PacBio platform (Pacific BioSciences). The 454 and Ion PGM platforms use emersion PCR for clonal amplification, and the HiSeq platform uses Bridge amplification. In the sequencing-by-synthesis method, sequencing is performed by detecting phosphate, hydrogen ion, or fluorescence, which is generated when DNA is synthesized by sequentially adding single nucleotides. In the process of detecting sequences, the 454 platform is based on pyrosequencing, and the Ion PGM platform is based on the detection of hydrogen ion. The HiSeq and PacBio platforms perform sequencing by detecting fluorescence.

The sequencing-by-ligation method is a sequencing technique that uses DNA ligase, and is performed to identify a nucleotide present at a specific position of a DNA nucleotide sequence. Unlike most sequencing techniques that use polymerase, the sequencing-by-ligation method is characterized in that polymerase is not used and DNA ligase does not ligate mismatched sequences. The SOLiD system corresponds to this method. In this technique, two nucleotides are read at every step of the sequencing process. The reading is individually repeated five times by primer reset, and thus each nucleotide is read twice, making the data highly accurate.

In the sequencing-by-ligation method, among dinucleotide primer sets made of 16 combinations, dinucleotide primers corresponding to the nucleotide sequence of interest are sequentially ligated, and a combination of the ligations is finally analyzed to determine the nucleotide sequence of the DNA of interest.

### Sequencing, sequencing-by-synthesis or sequencing-by-ligation that use methylated DNA-specific binding protein or antibody

In a sequencing or next-generation sequencing method that uses a methylated DNA-specific binding protein or antibody, a protein or antibody that binds specifically to methylated DNA is mixed with DNA, and then it binds specifically to methylated DNA. Thus, only methylated DNA can be selectively isolated. In the present invention, genomic DNA was mixed with a methylated DNA-specific binding protein, and then only methylated DNA was selectively isolated. The isolated DNA was amplified using PCR primers, and then whether the DNA was methylated was measured by the Sanger method or the sequencing-by-synthesis or sequencing-by-ligation method.

Herein, the next-generation sequencing method may be performed by the sequencing-by-synthesis or sequencing-by-ligation method. In addition, the methylated DNA-specific binding protein may be MBD2bt, but is not limited thereto, and the antibody may be 5'-methyl-cytosine, but is not limited thereto.

### Kit

Disclosed is a kit useful for the detection of a cellular proliferative disorder in a subject. The kit comprises a carrier means compartmentalized to receive a sample therein, one or more containers comprising a second container containing PCR primers for amplification of a 5'-CpG-3' base sequence, and a third container containing a sequencing primer for pyrosequencing an amplified PCR product.

Carrier means are suited for containing one or more containers such as vials, tubes, and the like, each of the containers comprising one of the separate elements to be used in the method. In view of the description provided herein of the inventive use, those of skill in the art can readily determine the apportionment of the necessary reagents among the containers.

### Substrates

After the target nucleic acid region has been amplified, the nucleic acid amplification product can be hybridized to a known gene probe attached to a solid support (substrate) to detect the presence of the nucleic acid sequence.

As used herein, the term "substrate", when used in reference to a substance, structure, surface or material, means a composition comprising a nonbiological, synthetic, nonliving, planar or round surface that is not heretofore known to comprise a specific binding, hybridization or catalytic recognition site or a plurality of different recognition sites or a number of different recognition sites which exceeds the number of different molecular species comprising the surface, structure or material. Examples of the substrate include, but are not limited to, semiconductors, synthetic (organic) metals, synthetic semiconductors, insulators and dopants; metals, alloys, elements, compounds and minerals; synthetic, cleaved, etched, lithographed, printed, machined and microfabricated slides, devices, structures and surfaces; industrial polymers, plastics, membranes silicon, silicates, glass, metals and ceramics; and wood, paper, cardboard, cotton, wool, cloth, woven and nonwoven fibers, materials and fabrics; and amphibious surfaces.

It is known in the art that several types of membranes have adhesion to nucleic acid sequences. Specific non-limiting examples of these membranes include nitrocellulose or other membranes used for detection of gene expression such as polyvinylchloride, diazotized paper and other commercially available membranes such as GENESCREEN™, ZETAPROBE™ (Biorad) and NYTRAN™. Beads, glass, wafer and metal substrates are also included. Methods for attaching nucleic acids to these objects are well known in the art. Alternatively, screening can be done in a liquid phase.

### Hybridization Conditions

In nucleic acid hybridization reactions, the conditions used to achieve a particular level of stringency will vary depending on the nature of the nucleic acids being hybridized. For example, the length, degree of complementarity, nucleotide sequence composition (e.g., GC/AT content), and nucleic acid type (e.g., RNA/DNA) of the hybridizing regions of the nucleic acids can be considered in selecting hybridization conditions. An additional consideration is whether one of the nucleic acids is immobilized, for example, on a filter.

An example of progressively higher stringency conditions is as follows: 2X SSC/0.1% SDS at room temperature (hybridization conditions); 0.2X SSC/0.1% SDS at room temperature (low stringency conditions); 0.2X SSC/0.1% SDS at 42 °C (moderate stringency conditions); and 0.1X SSC at about 68 °C (high stringency conditions). Washing can be carried out using only one of these conditions, e.g., high stringency conditions, or each of the conditions can be used, e.g., for 10-15 minutes each, in the order listed above, repeating any or all of the steps listed. However, as mentioned above, optimal conditions will vary depending on the particular hybridization reaction involved, and can be determined empirically. In general, conditions of high stringency are used for the hybridization of the probe of interest.

### Label

The probe of interest can be detectably labeled, for example, with a radioisotope, a fluorescent compound, a bioluminescent compound, a chemiluminescent compound, a metal chelator, or an enzyme. Appropriate labeling with such probes is widely known in the art and can be performed by any conventional method.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Measurement of methylation of SDC2 biomarker gene in tissue of gastric polyp patients

In order to evaluate the usefulness of the *SDC2* biomarker gene for early diagnosis of gastric polyp that is a precancerous lesion, genomic DNA was isolated from normal gastric tissues (Biochain; 5 samples) and the paraffin tissues of gastric polyp patients (provided by the Biobank of the Chungnam National University Hospital; 10 low-grade dysplasia samples, and 10 high-grade dysplasia samples). Isolation of genomic DNA from the paraffin tissues was performed using a QIAamp DNA Micro Kit (Qiagen, Germany) according to the manufacturer's instruction.

Measurement of methylation was performed using a quantitative pyrosequencing method. 200 ng of the isolated genomic DNA was treated with bisulfite using an EZ DNA methylation-Gold kit (Zymo Research, USA). When the DNA was treated with bisulfite, unmethylated cytosine was modified to uracil, and methylated cytosine remained without changes. The DNA treated with bisulfite was eluted with 20 *µ*ℓ of sterile distilled water and subjected to pyrosequencing. PCR and sequencing primers for performing pyrosequencing for the SDC2gene were designed using PSQ assay design program (Qiagen, Germany). The PCR and sequencing primers for measuring the methylation of each gene are shown in Table 1.

**Table 1: Primers for bisulfite-PCR and pyrosequencing**

| Genes | Primers | Sequences (5'-> 3')^{a} | SEQ ID NO: | CpG location^{b} | Size of amplicon (bp) |
|---|---|---|---|---|---|
| *SDC2* | Forward | GGGAGTGTYGAAATTAATAAGTG | 2 | +460, +466, +473, +479 | 149 |
| | Reverse | Biotin-ACCAAAACAAAACRAAACCTCCTACCCA | 3 | | |
| | Sequencing | AGGAGGAGGAAGYGAG | 4 | | |

| | | | | | |
|---|---|---|---|---|---|
| ^{a} Y = C or T; R=A or G ^{b} distances (nucleotides) from the transcription start site (+1): the positions of CpG regions on the genomic DNA used in the measurement of methylation | | | | | |

20 ng of the genomic DNA treated with bisulfite was amplified by PCR. In the PCR amplification, a PCR reaction solution (20 ng of the genomic DNA treated with bisulfite, 5 *µ*ℓ of 10×PCR buffer (Enzynomics, Korea), 5 units of Taq polymerase (Enzynomics, Korea), 4 *µ*ℓ of 2.5 mM dNTP (Solgent, Korea), and 2 *µ*ℓ (10 pmole/µl) of PCR primers) was used, and the PCR reaction was performed under the following conditions: predenaturation at 95□ for 5 min, and then 45 cycles of denaturation at 95□ for 40 sec, annealing at 60□ for 45 sec and extension at 72□ for 40 sec, followed by final extension at 72□ for 5 min. The amplification of the PCR product was confirmed by electrophoresis on 2.0% agarose gel.

The amplified PCR product was treated with PyroGold reagents (Biotage, USA), and then subjected to pyrosequencing using the PSQ96MA system (Biotage, USA) according to the manufacturer's instruction. After the pyrosequencing, the methylation level of the DNA was measured by calculating the methylation index. The methylation index was calculated by determining the average rate of cytosine binding to each CpG island.

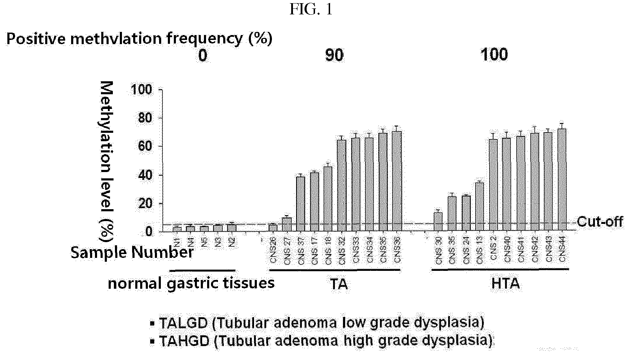

As a result, as can be seen in FIG. 1, the normal gastric tissues showed no methylation, and the low-grade dysplasia showed a methylation frequency of 90.0% (9/10), and the high-grade dysplasia showed a methylation frequency of 100% (10/10).

Such results indicate that the *SDC2* biomarker gene showed a high level and frequency of methylation even in gastric polyp that is a precancerous lesion of gastric cancer, suggesting that the *SDC2* biomarker gene is useful as a biomarker for diagnosis of gastric polyp and early diagnosis of gastric cancer.

### Example 2: Measurement of methylation of biomarker gene in gastric cancer cell line and gastric cancer tissue

In order to examine whether the *SDC2* biomarker gene confirmed to be methylated in gastric polyp is also useful as a biomarker for diagnosis of gastric cancer, pyrosequencing was performed in the same manner as described in Example 1.

Genomic DNA was isolated from the gastric cancer cell line AGS (Korean Cell Line Bank (KCLB No. 21739)), and the cancer tissues of 41 gastric cancer patients and normal tissues adjacent to the cancer tissues (provided by the Biobank of the Chungnam National University Hospital) using a QIAamp DNA mini Kit (Qiagen, Germany) according to the manufacturer's instruction.

200 ng of the isolated genomic DNA was treated with bisulfite using an EZ DNA methylation-Gold kit (Zymo Research, USA). When the genomic DNA was treated with bisulfite, unmethylated cytosine was modified to uracil, and methylated cytosine remained without changes. The DNA treated with bisulfite was eluted with 20 *µ*ℓ of sterile distilled water and subjected to pyrosequencing.

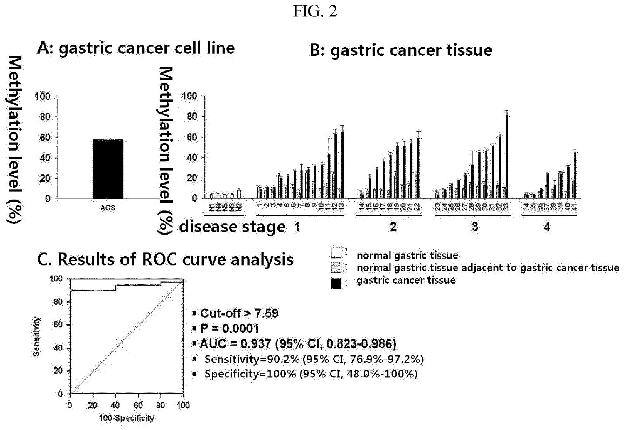

FIG. 2A shows the results of quantitatively measuring the methylation levels of the *SDC2* biomarker gene in the gastric cancer cell line by the pyrosequencing method. As can be seen therein, the *SDC2* biomarker gene was expressed at a high level in the gastric cancer cell line AGS. This suggests that the *SDC2* gene shows a high level of methylation in the gastric cancer cell line, indicating that the *SDC2* gene is useful as a biomarker for diagnosis of gastric cancer.

In order to verify this suggestion, an experiment on the verification of methylation of the *SDC2* gene in a gastric cancer tissue sample was performed.

To verify the methylation of the *SDC2* gene in gastric cancer tissue, the methylation levels of the *SDC2* gene in gastric cancer tissues at various disease stages (disease stage 1: 13 persons; disease stage 2: 9 persons; disease stage 3: 11 persons; and disease stage 4: 8 persons) were measured in the same manner as described in Example 1.

As a result, as shown in FIG. 2B, the methylation level of the *SDC2* gene in the gastric cancer tissue was significantly higher than those in the gastric tissue of the normal persons and the normal gastric tissue adjacent to the gastric cancer tissue.

In addition, in order to evaluate the sensitivity and specificity of the *SDC2* gene for gastric cancer diagnosis, ROC curve analysis was performed. As a result, the gene showed a high sensitivity of 90.2% and a very high specificity of 100% (FIG. 2C). Such results indicate that the *SDC2* methylation biomarker gene is useful for gastric cancer diagnosis.

### Example 3: Measurement of methylation of SDC2 biomarker gene in sera of gastric cancer patients

In order to examine the usefulness of the *SDC2* biomarker gene as a biomarker for gastric cancer diagnosis using serum, the methylation of the *SDC2* biomarker gene in the sera of gastric cancer patients was measured by a quantitative methylation-specific real time PCR (qMSP) method.

For this purpose, two PCR primers (IDT, USA) capable of specifically amplifying methylated *SDC2* gene treated with bisulfite, and a fluorescent probe (IDT, USA), were designed. To determine the amount and quality of serum DNA treated with bisulfite, *ACTB* gene was used as an internal control. The sequences of the PCR primers and fluorescent probe used in qMSP are shown in Table 2 below.

**Table 2: Sequences of primers and fluorescent probe for qMSP**

| Gene | Sequences (5'→ 3') | SEQ ID NO: | Size of amplified product (bp) |
|---|---|---|---|
| *SDC2* | Forward: TAGAAATTAATAAGTGAGAGGGCGT | 5 | 121 |
| | Reverse: GACTCAAACTCGAAAACTCGAA | 6 | |
| | Fluorescent probe: FAM-AGTAGGCGTAGGAGGAGGAAGCGA - Iowa Black | 7 | |
| *ACTB* | Forward: TGGTGATGGAGGAGGTTTAGTAAGT | 20 | 136 |
| | Reverse: AACCAATAAAACCTACTCCTCCCTTAA | 21 | |
| | Fluorescent probe: TET-ACCACCACCCAACACACAATAACAAACACA - Iowa Black | 22 | |

Genomic DNA was isolated from 800 ul of serum using a DynalBeads SILANE viral NA kit (Invitrogen) according to the manufacturer's instruction. The isolated genomic DNA was treated with bisulfite using an EZ DNA methylation-Gold kit (Zymo Research, USA), and then eluted with 20 *µ*ℓ of sterile distilled water and used in qMSP. 1/3 of the volume of the genomic DNA treated with bisulfite used in qMSP. Real-time PCR was performed using a Rotor Gene-Q Real Time PCR system (Qiagen, Germany) with a Rotor Gene Probe Kit (Qiagen, Germany). A final volume of 20 *µ*ℓ was subjected to real-time PCR under the following conditions: for *SDC2,* 10 min at 95□, and 50 cycles, each consisting of 10 sec at 95□, 1 sec at 62□ and 20 sec at 72□; for *ACTB,* 10 min at 95□, and 50 cycles, each consisting of 10 sec at 95□, 60 sec at 58□. The methylation level was measured as the Percentage of Methylated Reference (PMR) by a Comparative Cycle Threshold (Ct) method, and the artificially methylated genomic DNA of the gastric cancer cell line AGS (Korean Cell Line Bank (KCLB No. 21739)) was used as a reference. The PMR was calculated using the following equation: PMR = 2^{-ΔΔCt}X100; ΔΔCt=[(*C*_{t*SDC2*}-*C*_{t*ACTB*})sample]-[(*C*ₜ*_{SDC2}-*C_{t*ACTB*})AGS].

To evaluate the ability of the *SDC2* biomarker gene to diagnose gastric cancer in serum, DNA in the sera of 130 normal persons and 40 gastric cancer patients was subjected to qMSP.

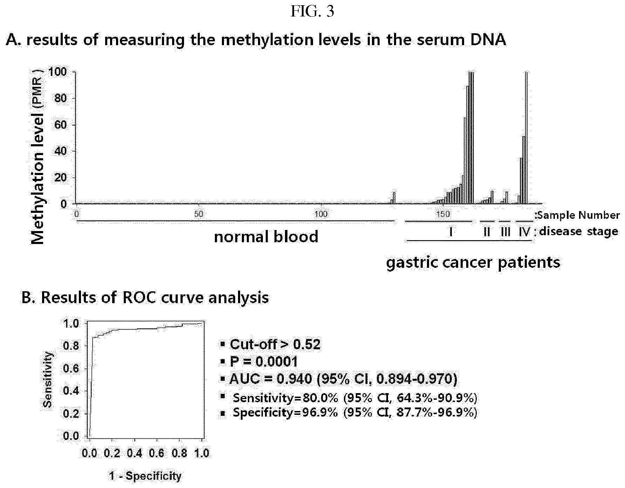

As a result, as can be seen in FIG. 3A, the sera of the normal persons showed little or no methylation of the *SDC2* biomarker gene, and the sera of the gastric cancer patients showed methylation of the *SDC2* biomarker gene at a high level and frequency. Particularly, the sera at disease stages 1 and 2 of gastric cancer showed a high level and frequency of methylation of the *SDC2* biomarker gene.

To measure the sensitivity and specificity of the *SDC2* biomarker gene for gastric cancer diagnosis, ROC curve (Receiver Operating Characteristic) analysis was performed using MedCalc program (MEDCALC, Belgium).

As a result, as shown in FIG. 3B, the *SDC2* gene showed a sensitivity and specificity of 80.0% and 96.9%, respectively, suggesting that it has a very high ability to diagnose gastric cancer. Particularly, the *SDC2* gene showed a sensitivity of 80.0% for early-stage gastric cancer or advanced gastric cancer, suggesting that it is useful for early diagnosis of gastric cancer.

### Example 4: Measurement of methylation of SDC2 biomarker genes in other solid cancer tissues

In order to examine whether the *SDC2* biomarker gene can be specifically used as a gastric polyp- and gastric cancer-specific diagnostic marker, experiments on other kinds of cancer were performed. In order for the *SDC2* biomarker gene to be used as a marker for diagnosis of gastric polyp or gastric cancer, it should not be methylated in various organ tissues of normal persons other than patients and in other solid cancer tissues.

To verify whether the *SDC2* biomarker gene satisfies this requirement, genomic DNA was separated from various organic tissues (Biochain) of normal persons other than patients, various solid cancer tissues and normal tissues adjacent to the cancer tissues (provided by the Biobank of the Chungnam National University Hospital) using a QIAamp DNA Mini kit (QIAGEN, USA). 200 ng of the isolated genomic DNA was treated with bisulfite using an EZ DNA methylation-Gold kit (Zymo Research, USA), and then eluted with 20 *µ*ℓ of sterile distilled water and used in pyrosequencing.

20 ng of the genomic DNA treated with bisulfite was amplified by PCR. In the PCR amplification, a PCR reaction solution (20 ng of the genomic DNA treated with bisulfite, 5 *µ*ℓ of 10×PCR buffer (Enzynomics, Korea), 5 units of Taq polymerase (Enzynomics, Korea), 4 *µ*ℓ of 2.5 mM dNTP (Solgent, Korea), and 2 *µ*ℓ (10 pmole/µl) of PCR primers) was used, and the PCR reaction was performed under the following conditions: predenaturation at 95□ for 5 min, and then 45 cycles of denaturation at 95□ for 40 sec, annealing at 60D□ for 45 sec and extension at 72□ for 40 sec, followed by final extension at 72□ for 5 min. The amplification of the PCR product was confirmed by electrophoresis on 2.0% agarose gel.

The amplified PCR product was treated with PyroGold reagents (Biotage, USA), and then subjected to pyrosequencing using the PSQ96MA system (Biotage, USA). After the pyrosequencing, the methylation level of the *SDC2* biomarker gene was measured by calculating the methylation index. The methylation index was calculated by determining the average rate of cytosine binding to each CpG island.

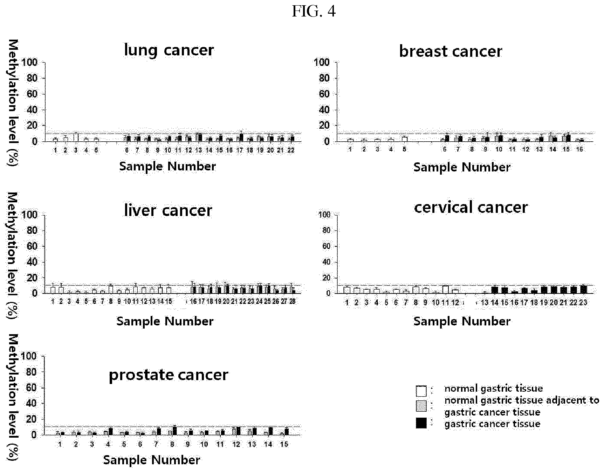

As a result, as can be seen in FIG. 4, the methylation level of the *SDC2* biomarker gene was 10% or lower in all of lung cancer tissue, breast cancer tissue, liver cancer tissue, cervical cancer tissue and prostate cancer tissue. This suggests that the *SDC2* biomarker gene is methylated specifically in gastric cancer tissue. Such results indicate that the *SDC2* biomarker gene can be used as a biomarker not only for gastric cancer-specific diagnosis, but also for diagnosis of bowel cancer.

### Example 5: Measurement of methylation of SDC2 biomarker gene in sera of gastric polyp patients

In order to confirm the usefulness of the *SDC2* biomarker gene for diagnosis of gastric polyp in serum, the methylation of the *SDC2* biomarker gene in the sera of gastric polyp patients was measured by a quantitative methylation-specific real time PCR (qMSP) method. qMSP was performed in the same manner as described in Example 3.

To evaluate the ability of the *SDC2* biomarker gene to diagnose gastric polyp in serum, qMSP was performed on DNA in the sera of 12 normal persons determined to be normal by gastroscopy, 5 hyperplastic polyp patients and 16 adenomatous polyp patients. The methylation level was expressed as the cycle threshold (Ct) of the *SDC2* gene, and when the Ct value was not formed because methylation was not methylated, the Ct value was expressed as 40.

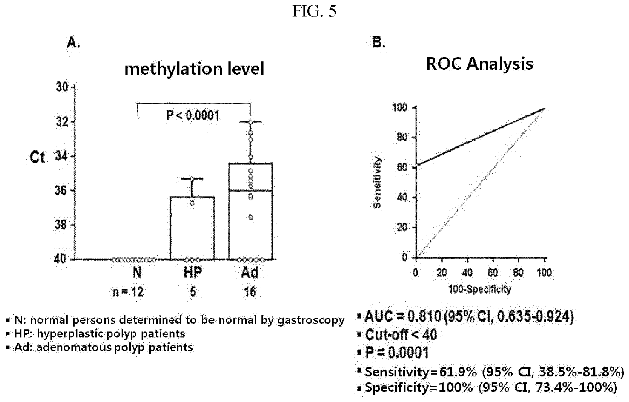

As a result, as can be seen in FIG. 5A, the sera of the 12 normal person showed no methylation of the *SDC2* gene, and the sera of the gastric polyp patients showed methylation of the *SDC2* gene at a high level and frequency.

To measure the sensitivity and specificity of the *SDC2* biomarker gene for gastric polyp diagnosis, ROC curve (Receiver Operating Characteristic) analysis was performed using MedCalc program (MEDCALC, Belgium).

As a result, as can be seen in FIG. 5B, the *SDC2* gene showed a sensitivity and specificity of 61.9% (13/21) and 100%, respectively, for the total gastric polyp patients, suggesting that it has an excellent ability to diagnose gastric polyp. In addition, the *SDC2* gene showed a sensitivity of 40% (2/5) for hyperplastic polyp and a sensitivity of 68.8% (11/16) for adenomatous polyp. Thus, it was found that the *SDC2* gene is highly useful for diagnosis of gastric polyp in blood.

### INDUSTRIAL APPLICABILITY

As described above, the present invention has an effect in that the methylation of the CpG island of the gastric polyp- and gastric cancer-specific marker gene can be detected to thereby provide information for diagnosing gastric cancer.

The use of the methylated CpG island of syndecan-2 (*SDC2*) gene, located in a region set forth in SEQ ID NO: 1, according to the present invention or the methylation detection method or the diagnostic composition, kit or nucleic acid chip disclosed makes it possible to diagnose gastric cancer at an early transformation stage, thus enabling the early diagnosis of gastric cancer. In addition, the method disclosed enables gastric cancer to be effectively diagnosed in an accurate and rapid manner compared to conventional methods.
## Claims

1. Use of a methylated CpG island of syndecan-2 *(SDC2)* gene located in a region set forth in SEQ ID NO: 1 for diagnosing gastric cancer or gastric polyp.

2. The use of claim 1, wherein the CpG island is located in any one of the promoter region, 5'-untranslated region, the exon, and the intron of the *SDC2* gene.

3. The use of claim 1, wherein the methylated CpG island is detected by any one selected from the group consisting of a primer pair capable of amplifying a fragment comprising the methylated CpG island, a probe capable of hybridizing to the methylated CpG island, a sequencing primer, a sequencing-by-synthesis primer, and a sequencing-by-ligation primer.

4. The use of claim 3, wherein the methylated CpG island is detected by a primer pair capable of amplifying a fragment comprising the methylated CpG island.

5. The use of claim 4, wherein the methylated CpG island is detected after modifying the fragment comprising the CpG island with the treatment by a reagent that modifies a methylated DNA and a non-methylated DNA differently.

6. The use of claim 4, wherein the primer pair is set forth in SEQ ID NO: 2 and 3 or set forth in SEQ ID NO: 5 and 6.

7. The use of claim 4, further comprising a sequencing primer comprising sequence set forth in SEQ ID NO: 4.

8. The use of claim 3, wherein the methylated CpG island is detected by a probe capable of hybridizing to the methylated CpG island.

9. The use of claim 8, wherein the probe is selected from the group consisting of the base sequences set forth in SEQ ID NOS: 8 to 19.

## Patentansprüche

1. Verwendung einer methylierten CpG-Insel des Gens Syndecan-2 *(SDC2),* die in einem Bereich, dargelegt durch SEQ ID NO: 1, lokalisiert ist, um ein Magenkarzinom oder Magenpolypen zu diagnostizieren.

2. Verwendung nach Anspruch 1, wobei die CpG-Insel in irgendeinem von dem Promotorbereich, 5'-untranslatiertem Bereich, dem Exon und dem Intron des Gens *SDC2* lokalisiert ist.

3. Verwendung nach Anspruch 1, wobei die methylierte CpG-Insel durch irgendwas ausgewählt aus der Gruppe, bestehend aus einem Primerpaar, das in der Lage ist, ein Fragment, umfassend die methylierte CpG-Insel, zu amplifizieren, einer Sonde, die in der Lage ist, mit der methylierten CpG-Insel zu hybridisieren, einem Sequenzierungsprimer, einem Sequenzierung-durch-Synthese-Primer und einem Sequenzierung-durch-Ligation-Primer, detektiert wird.

4. Verwendung nach Anspruch 3, wobei die methylierte CpG-Insel durch ein Primerpaar, das in der Lage ist, ein Fragment, umfassend die methylierte CpG-Insel, zu amplifizieren, detektiert wird.

5. Verwendung nach Anspruch 4, wobei die methylierte CpG-Insel detektiert wird, nachdem das Fragment, umfassend die CpG-Insel, durch Behandlung mit einem Reagenz modifiziert wird, das eine methylierte DNA und eine nicht methylierte DNA unterschiedlich modifiziert.

6. Verwendung nach Anspruch 4, wobei das Primerpaar in SEQ ID NO: 2 und 3 dargelegt ist oder in SEQ ID NO: 5 und 6 dargelegt ist.

7. Verwendung nach Anspruch 4, ferner umfassend einen Sequenzierungsprimer, umfassend eine in SEQ ID NO: 4 dargelegte Sequenz.

8. Verwendung nach Anspruch 3, wobei die methylierte CpG-Insel durch eine Sonde detektiert wird, die in der Lage ist, mit der methylierten CpG-Insel zu hybridisieren.

9. Verwendung nach Anspruch 8, wobei die Sonde ausgewählt ist aus der Gruppe, bestehend aus den in SEQ ID NOS: 8 bis 19 dargelegten Basensequenzen.

## Revendications

1. Utilisation d'un îlot CpG méthylé du gène de syndécane 2 (*SDC2*) localisé dans une région représentée par SEQ ID NO : 1 pour le diagnostic de cancer gastrique ou de polype gastrique.

2. Utilisation selon la revendication 1, dans laquelle l'îlot CpG est localisé dans l'une quelconque de la région promoteur, la région non traduite en 5', l'exon, et l'intron du gène *SDC2.*

3. Utilisation selon la revendication 1, dans laquelle l'îlot CpG méthylé est détecté par l'une quelconque choisie dans le groupe constitué d'une paire d'amorces capable d'amplifier un fragment comprenant l'îlot CpG méthylé, une sonde capable de s'hybrider à l'îlot CpG méthylé, une amorce de séquençage, une amorce de séquençage par synthèse, et une amorce de séquençage par ligature.

4. Utilisation selon la revendication 3, dans laquelle l'îlot CpG méthylé est détecté par une paire d'amorces capable d'amplifier un fragment comprenant l'îlot CpG méthylé.

5. Utilisation selon la revendication 4, dans laquelle l'îlot CpG méthylé est détecté après modification du fragment comprenant l'îlot CpG avec le traitement par un réactif qui modifie un ADN méthylé et un ADN non méthylé de façon différente.

6. Utilisation selon la revendication 4, dans laquelle la paire d'amorces est représentée par SEQ ID NO : 2 et 3 ou représentée par SEQ ID NO : 5 et 6.

7. Utilisation selon la revendication 4, comprenant en outre une amorce de séquençage comprenant la séquence représentée par SEQ ID NO : 4.

8. Utilisation selon la revendication 3, dans laquelle l'îlot CpG méthylé est détecté par une sonde capable de s'hybrider à l'îlot CpG méthylé.

9. Utilisation selon la revendication 8, dans laquelle la sonde est choisie dans le groupe constitué des séquences de bases représentées par SEQ ID NO : 8 à 19.


<110> GENOMICTREE, INC.
<120> Method for Detecting Gastric Polyp and Gastric Cancer Using Gastric Polyp and Gastric Cancer Specific Methylation Marker Gene
<130> PP-B1257
<150> KR10-2012-0125539
   <151> 2012-11-07
<160> 23
<170> KopatentIn 2.0
<210> 1
   <211> 2000
   <212> DNA
   <213> Promoter, 5' untranslated region and 1st intron of SDC2 gene
<400> 1
   ttgggggtgc ggaaggattt gggagaatgg gaaacactct cactatatat ttattacatt      60
   aattatcttc tcttttaaaa tgcaattttc atgaactggc gatttatgaa cacttcacat     120
   tgcttgaaag catcttacac tttttttttc cctcaactca caaagcagtt tctttctact     180
   ggtcgaattc tcaaggcaga aaagctacat acgtctctcg tttcttcact aattgttctc     240
   tagaaaaggg aaagtgaaga agggaaagag aaaagacaac ggggaagaaa agagcataga     300
   ggagagagga aaagtgggga gagaaaggaa gaaaaggact gagaaaacgc aggagccctg     360
   gcttgccggt gagcagagcc ggcgcagcca cagcgcggag ccgcggcgcc cactggtcct     420
   cggagctgcc aatcggcgtg taatcctgta ggaatttctc ccgggtttat ctgggagtca     480
   cactgccgcc tcctctcccc agtcgcccag gggagcccgg agaagcaggc tcaggaggga     540
   gggagccaga ggaaaagaag aggaggagaa ggaggaggac ccggggaggg aggcgcggcg     600
   cgggaggagg aggggcgcag ccgcggagcc agtggccccg cttggacgcg ctgctctcca     660
   gatacccccg gagctccagc cgcgcggatc gcgcgctccc gccgctctgc ccctaaactt      720
   ctgccgtagc tccctttcaa gccagcgaat ttattcctta aaaccagaaa ctgaacctcg     780
   gcacgggaaa ggagtccgcg gaggagcaaa accacagcag agcaagaaga gcttcagaga     840
   gcagccttcc cggagcacca actccgtgtc gggagtgcag aaaccaacaa gtgagagggc     900
   gccgcgttcc cggggcgcag ctgcgggcgg cgggagcagg cgcaggagga ggaagcgagc     960
   gcccccgagc cccgagcccg agtccccgag cctgagccgc aatcgctgcg gtactctgct    1020
   ccggattcgt gtgcgcgggc tgcgccgagc gctgggcagg aggcttcgtt ttgccctggt    1080
   tgcaagcagc ggctgggagc agccggtccc tggggaatat gcggcgcgcg tggatcctgc    1140
   tcaccttggg cttggtggcc tgcgtgtcgg cggagtcggt gagtgggcca ggcggaggat    1200
   gcgcgcgccg tttagggtgt ttgaagctac gagaggagcc cgcagggaat aggggagcgc    1260
   cacctgggga acccccagtc cccaagtata caccggagat ccgctgggac aaatgcgctc    1320
   gtccggtcac cctttccccc tcttcccttc ctcagaaaag cgctgctcgc tggcgttacc 1380
   ccgcggtccg cgggaatggg ggcaccgaga attgcggttt ggtctagccg cagaggcccc 1440
   tgaagtcact cccaacttct tcgccctcgg cgggtcttgc tgcgtggtct gggaaggacg 1500
   gaggggaaag ggtggcagga ggggggagcc tgggtcgggc ccgcgaggga acggctccac 1560
   tccgcgcgct cctcgagacc agggatgacc tggaaacttc ggggtccctt cctccgcaca 1620
   ccatcccccc cgcgccagct ttcctgtttg actgcatgca agttctgggg agatgggggc 1680
   cagatttaag agacccgcga gtgtccagag agaaaagttt gcaaaagttc ttttgtttga 1740
   tgctccctgc ggctagggcg aggtaaccga cactacgtgg aatcgcagta ggcgatccct 1800
   caaggggata ctgggggagg cacggaacgc gtccgaaaat gctgggacgc cggccactgg 1860
   attcccagtc ctgcggcgac cccctcctcg ttgaggggtg gaggttgcac cgcggggcgt 1920
   cagggacggg aggacatttt cataggagtt acacgggagt gccgcaagca gggcgaggcg 1980
   gggtacgtgt gacacggcgc 2000
<210> 2
   <211> 23
   <212> DNA
   <213> Artificial Sequence
<220>
   <223> Forward primer of SDC2 gene
<400> 2
   gggagtgtyg aaattaataa gtg 23
<210> 3
   <211> 28
   <212> DNA
   <213> Artificial Sequence
<220>
   <223> Backward primer of SDC2 gene
<400> 3
   accaaaacaa aacraaacct cctaccca 28
<210> 4
   <211> 16
   <212> DNA
   <213> Artificial Sequence
<220>
   <223> Sequencing primer of SDC2 gene
<400> 4
   aggaggagga agygag 16
<210> 5
   <211> 25
   <212> DNA
   <213> Artificial Sequence
<220>
   <223> Forward primer of SDC2 gene
<400> 5
   tagaaattaa taagtgagag ggcgt 25
<210> 6
   <211> 22
   <212> DNA
   <213> Artificial Sequence
<220>
   <223> Backward primer of SDC2 gene
<400> 6
   gactcaaact cgaaaactcg aa 22
<210> 7
   <211> 24
   <212> DNA
   <213> Artificial Sequence
<220>
   <223> Flourescene probe of SDC2 gene
<400> 7
   agtaggcgta ggaggaggaa gcga 24
<210> 8
   <211> 30
   <212> DNA
   <213> Artificial Sequence
<220>
   <223> Probe for SDC2 gene
<400> 8
   cggagctgcc aatcggcgtg taatcctgta 30
<210> 9
   <211> 50
   <212> DNA
   <213> Artificial Sequence
<220>
   <223> Probe for SDC2 gene
<400> 9
   ctgccgtagc tccctttcaa gccagcgaat ttattcctta aaaccagaaa 50
<210> 10
   <211> 50
   <212> DNA
   <213> Artificial Sequence
<220>
   <223> Probe for SDC2 gene
<400> 10
   gcacgggaaa ggagtccgcg gaggagcaaa accacagcag agcaagaaga 50
<210> 11
   <211> 60
   <212> DNA
   <213> Artificial Sequence
<220>
   <223> Probe for SDC2 gene
<400> 11
   gcagccttcc cggagcacca actccgtgtc gggagtgcag aaaccaacaa gtgagagggc 60 60
<210> 12
   <211> 50
   <212> DNA
   <213> Artificial Sequence
<220>
   <223> Probe for SDC2 gene
<400> 12
   cccgagcccg agtccccgag cctgagccgc aatcgctgcg gtactctgct 50
<210> 13
   <211> 40
   <212> DNA
   <213> Artificial Sequence
<220>
   <223> Probe for SDC2 gene
<400> 13
   cttggtggcc tgcgtgtcgg cggagtcggt gagtgggcca 40
<210> 14
   <211> 30
   <212> DNA
   <213> Artificial Sequence
<220>
   <223> probe
<400> 14
   cggagttgtt aatcggcgtg taattttgta 30
<210> 15
   <211> 50
   <212> DNA
   <213> Artificial Sequence
<220>
   <223> probe
<400> 15
   ttgtcgtagt ttttttttaa gttagcgaat ttatttttta aaattagaaa 50
<210> 16
   <211> 50
   <212> DNA
   <213> Artificial Sequence
<220>
   <223> probe
<400> 16
   gtacgggaaa ggagttcgcg gaggagtaaa attatagtag agtaagaaga 50
<210> 17
   <211> 60
   <212> DNA
   <213> Artificial Sequence
<220>
   <223> probe
<400> 17
   gtagtttttt cggagtatta atttcgtgtc gggagtgtag aaattaataa gtgagagggt 60 60
<210> 18
   <211> 50
   <212> DNA
   <213> Artificial Sequence
<220>
   <223> probe
<400> 18
   ttcgagttcg agttttcgag tttgagtcgt aatcgttgcg gtattttgtt 50
<210> 19
   <211> 40
   <212> DNA
   <213> Artificial Sequence
<220>
   <223> probe
<400> 19
   tttggtggtt tgcgtgtcgg cggagtcggt gagtgggtta 40
<210> 20
   <211> 25
   <212> DNA
   <213> Artificial Sequence
<220>
   <223> Forward primer of ACTB gene
<400> 20
   tggtgatgga ggaggtttag taagt 25
<210> 21
   <211> 27
   <212> DNA
   <213> Artificial Sequence
<220>
   <223> Backward primer of ACTB gene
<400> 21
   aaccaataaa acctactcct cccttaa 27
<210> 22
   <211> 30
   <212> DNA
   <213> Artificial Sequence
<220>
   <223> Fluorescence probe of ACTB gene
<400> 22
   accaccaccc aacacacaat aacaaacaca 30
<210> 23
   <211> 2000
   <212> DNA
   <213> Artificial Sequence
<220>
   <223> Bisulfite treated promoter, 5' untranslated region and 1st intron of SDC2 gene
<400> 23
   ttgggggtgc ggaaggattt gggagaatgg gaaatatttt tattatatat ttattatatt 60
   aattattttt ttttttaaaa tgtaattttt atgaattggc gatttatgaa tattttatat 120
   tgtttgaaag tattttatat tttttttttt ttttaattta taaagtagtt tttttttatt 180
   ggtcgaattt ttaaggtaga aaagttatat acgtttttcg tttttttatt aattgttttt 240
   tagaaaaggg aaagtgaaga agggaaagag aaaagataac ggggaagaaa agagtataga 300
   ggagagagga aaagtgggga gagaaaggaa gaaaaggatt gagaaaacgt aggagttttg 360
   gtttgtcggt gagtagagtc ggcgtagtta tagcgcggag tcgcggcgtt tattggtttt 420
   cggagttgtt aatcggcgtg taattttgta ggaatttttt tcgggtttat ttgggagtta 480
   tattgtcgtt tttttttttt agtcgtttag gggagttcgg agaagtaggt ttaggaggga 540
   gggagttaga ggaaaagaag aggaggagaa ggaggaggat tcggggaggg aggcgcggcg 600
   cgggaggagg aggggcgtag tcgcggagtt agtggtttcg tttggacgcg ttgtttttta 660
   gatattttcg gagttttagt cgcgcggatc gcgcgttttc gtcgttttgt ttttaaattt 720
   ttgtcgtagt ttttttttaa gttagcgaat ttatttttta aaattagaaa ttgaatttcg 780
   gtacgggaaa ggagttcgcg gaggagtaaa attatagtag agtaagaaga gttttagaga 840
   gtagtttttt cggagtatta atttcgtgtc gggagtgtag aaattaataa gtgagagggc 900
   gtcgcgtttt cggggcgtag ttgcgggcgg cgggagtagg cgtaggagga ggaagcgagc 960
   gttttcgagt ttcgagttcg agttttcgag tttgagtcgt aatcgttgcg gtattttgtt 1020
   tcggattcgt gtgcgcgggt tgcgtcgagc gttgggtagg aggtttcgtt ttgttttggt 1080
   tgtaagtagc ggttgggagt agtcggtttt tggggaatat gcggcgcgcg tggattttgt 1140
   ttattttggg tttggtggtt tgcgtgtcgg cggagtcggt gagtgggtta ggcggaggat 1200
   gcgcgcgtcg tttagggtgt ttgaagttac gagaggagtt cgtagggaat aggggagcgt 1260
   tatttgggga atttttagtt tttaagtata tatcggagat tcgttgggat aaatgcgttc 1320
   gttcggttat tttttttttt tttttttttt tttagaaaag cgttgttcgt tggcgttatt 1380
   tcgcggttcg cgggaatggg ggtatcgaga attgcggttt ggtttagtcg tagaggtttt 1440
   tgaagttatt tttaattttt tcgttttcgg cgggttttgt tgcgtggttt gggaaggacg 1500
   gaggggaaag ggtggtagga ggggggagtt tgggtcgggt tcgcgaggga acggttttat 1560
   ttcgcgcgtt tttcgagatt agggatgatt tggaaatttc ggggtttttt ttttcgtata 1620
   ttattttttt cgcgttagtt tttttgtttg attgtatgta agttttgggg agatgggggt 1680
   tagatttaag agattcgcga gtgtttagag agaaaagttt gtaaaagttt ttttgtttga 1740
   tgttttttgc ggttagggcg aggtaatcga tattacgtgg aatcgtagta ggcgattttt 1800
   taaggggata ttgggggagg tacggaacgc gttcgaaaat gttgggacgt cggttattgg 1860
   atttttagtt ttgcggcgat ttttttttcg ttgaggggtg gaggttgtat cgcggggcgt 1920
   tagggacggg aggatatttt tataggagtt atacgggagt gtcgtaagta gggcgaggcg 1980
   gggtacgtgt gatacggcgt 2000